# EUROPÄISCHE PATENTANMELDUNG

(11) **EP 0 566 010 A1**
(43) Veröffentlichungstag der Anmeldung: **20.10.1993**
(21) Anmeldenummer: 93105721.0
(22) Anmeldetag: 07.04.1993
(51) Int. Cl.: C12Q 1/66, G01N 33/04, G01N 21/76

(54) **Verfahren und Analyse-Kit zur Bestimmung der bakteriellen Keimzahl in Molke und Molkeninhaltsstoffen**

(30) Priorität: 13.04.1992 EP 92106340
(71) Anmelder: BIOLAC GmbH Gesellschaft für industrielle Nutzung von Milchinhaltsstoffen, D-31097 Harbarnsen (DE)
(72) Erfinder: Denzler, Hans-Jörg, W-3394 Langelsheim (DE)
(74) Vertreter: Lauer, Dieter, Dr.

(57) **Zusammenfassung**

Beschrieben wird ein Verfahren und ein Analyse-Kit zur Bestimmung der bakteriellen Keimzahl in Molke und Molkeninhaltsstoffen unter Anwendung der Luciferase-Luciferin-Reaktion.

## Beschreibung

Die Erfindung betrifft ein Verfahren zur Bestimmung der bakteriellen Keimzahl in Molke und Molkeninhaltstoffen unter Anwendung der Luciferin-Luciferase-Biolumineszenz-Reaktion sowie ein Analyse-Kit zur Durchführung dieses Verfahrens.

Die Verarbeitung von Molke und Molkeninhaltsstoffen erfordert eine ständige Überwachung der mikrobiologischen Qualität der eingesetzten Rohstoffe, damit eine mikrobielle Kontamination der daraus hergestellten Endprodukte ausgeschlossen werden kann. In Molkereien und milchverarbeitenden Betrieben werden daher ständig vor, während und nach der Verarbeitung mikrobiologische Tests durchgeführt, mit denen die hergestellten Endprodukte und die verarbeiteten Ausgangsprodukte, beispielsweise Molke und aus Molke gewonnene Molkenprodukte wie Lactose oder Molkenproteinpulver, auf die Anwesenheit unerwünschter Keime untersucht werden. Hierbei müssen oft sehr geringe Keimdichten nachgewiesen werden.

Eine der bisherigen Methoden, mit der die Keimzahl in Molkereiprodukten bestimmt wurde, ist das sogenannte Koch'sche Plattenverfahren. Bei dieser Methode werden die zu untersuchenden Proben auf Agarplatten ausgestrichen und für ca. 72 Stunden bei 30 °C inkubiert. Aus der Anzahl der gewachsenen Kolonien auf den Agarplatten kann anschließend auf die Keimzahl in den zu untersuchenden Proben geschlossen werden. Diese Bestimmungsmethode ist nicht nur sehr zeit- sondern auch sehr kostenintensiv, da bis zur Auswertung der Agarplatten die entsprechende Rohstofflieferung für diesen Zeitraum kühl zwischengelagert werden muß. Weitere Nachteile dieser Methode sind, daß sich mit ihr nur recht ungenau Sporenbildner, wie z.B. der Bacillus cereus, nachweisen lassen bzw. bei Anwesenheit von Sporenbildnern das Auszählen der Kolonien sehr erschwert wird.

Aus der DE 28 31 559 ist ein Verfahren zur Bestimmung somatischer Zellen in Milch bekannt. Dieses Verfahren beruht auf der bekannten Luciferin-Luciferase-Reaktion, bei der auf enzymatischem Wege ATP (ATP = Adenosintriphosphat) zu AMP (AMP = Adenosinmonophosphat) umgesetzt wird. Die bei dieser Reaktion auftretende Biolumineszenz wird quantitativ gemessen, wobei die freiwerdende Lichtmenge proportional zu der in der Probe enthaltenen Menge an ATP ist. Da jede Zelle ATP enthält, läßt sich aus der ATP-Konzentration die Anzahl der somatischen Zellen aus der Korrelation zwischen der gemessenen Probe und einem bekannten Standard errechnen.

Der ATP-Gehalt ist für jede Zellart charakteristisch. So besitzen Bakterienzellen weit weniger ATP als die wesentlich größeren somatischen Zellen. Auch in Hefezellen ist der ATP-Gehalt weit höher als in Bakterienzellen.

Aufgabe der Erfindung war es, ein Verfahren zur Bestimmung der Keimzahl in Molke und Molkeninhaltsstoffen zur Verfügung zu stellen, mit dem sich die bakterielle Keimdichte schnell ermitteln läßt. Eine weitere Aufgabe der Erfindung war es, ein Verfahren zur Verfügung zu stellen, mit dem sich auch Sporenbildner in Molke und Molkeninhaltsstoffen schnell nachweisen lassen.

Es wurde nun eine Verfahren gefunden, mit dem sich bakterielle Keime in Molke und Molkeninhaltsstoffen mit Hilfe der Luciferin-Luciferase-Biolumineszenz-Methode nachweisen lassen. Unter Molke wird dabei auch Molkenkonzentrat und Dünnmolke verstanden. Molkeninhaltsstoffe im Sinne der Erfindung sind alle aus Molke gewinnbaren Inhaltsstoffe, insbesondere Lactose und Molkenprotein, beispielsweise Lactosepulver oder Molkenproteinpulver wie Molkenproteinkonzentratpulver.

Gegenstand der Erfindung ist ein Verfahren zur Bestimmung der bakteriellen Keimzahl in Molke oder Molkeninhaltsstoffen, bei dem man in der zu analysierenden Probe
a) einen pH-Wert von 7,0 bis 7,9 einstellt,
b) das bakterielle ATP durch ein lysierendes Agens aus den Bakterienzellen freisetzt,
c) ein Luciferin-Luciferase-Reagenz hinzufügt,
d) die während der Lucifease-Luciferin-Reaktion freigesetzte Biolumineszenz-Lichtmenge quantitativ bestimmt und aus dieser gemessenen Lichtmenge die in der zu analysierenden Probe enthaltende Menge ATP bestimmt.

In Verfahrenschritt a) wird in der zu analysierenden Probe ein pH-Wert im Bereich von 7,0 bis 7,9 eingestellt. Bevorzugt ist dabei ein pH-Wert im Bereich von 7,3 bis 7,5, insbesondere der pH-Wert 7,4. Üblicherweise erfolgt die pH-Werteinstellung bei Raumtemperatur.

Je nach zu analysierender Probe kann in Verfahrensschritt a) die pH-Werteinstellung in unterschiedlicher Weise erfolgen. Bei der Bestimmung der Keimzahl in Lactosepulver oder Molkenproteinpulver wird der pH-Wert beispielsweise dadurch eingestellt, daß das Lactose- oder Molkenproteinpulver in steriler isotonischer Lösung, insbesondere in steriler physiologischer Kochsalzlösung, z.B. in an sich bekannter Ringerlösung, gelöst wird. Vorteilhaft verwendet man zur Lösung von Lactosepulver oder Molkenproteinpulver Ringerlösung. Soll die Keimzahl in Milch oder Molke bestimmt werden, wird der pH-Wert zweckmäßigerweise mit einem mit sterilem Wasser angesetzten,in der biochemischen Analytik üblichen Puffer für den pH-Bereich 7,0 bis 8,0, z.b. HEPES-Puffer, in Verfahrensschritt a) eingestellt. Bevorzugt verwendet man einen HEPES-Puffer, insbesondere einen um den pH-Wert 7,5 pufferenden HEPES-Puffer mit einer Konzentration von 1 mol/l.

Die Freisetzung des bakteriellen ATP in Verfahrensschritt b) wird durch ein zur Lysis von Bakterienzellmembranen geeignetes Reagenz erreicht. Dies kann enzymatisch, z. B. durch Lysozym, beispielsweise mit einer Lösung aus Lysozym und ca. 1,5 Gew.-% Trichloressigsäure, oder chemisch, z. B. durch zur Solubilisierung von Bakterienmembranen geeignete Detergenzien, erfolgen. Vorzugsweise setzt man ein sogenanntes an sich bekanntes NRB-Reagenz (NRB = "nucleotid releasing reagent for microbial cells") ein, das ein ionisches Detergenz in einer Konzentration von ca. 0,1 bis 2 Gew.-% enthält.

In Verfahrensschritt c) wird ein Luciferin-Luciferase-Reagenz eingesetzt, wie es aus zur Biolumineszenz befähigten Organismen gewonnen werden kann. Insbesondere verwendet man aus Glühwürmchen gewonnenes Luciferin-Luciferase-Reagenz, wie es allgemeim kommerziell erhältlich ist.

Zur Messung der Biolumineszenz-Lichtmenge in Verfahrensschritt d) können kommerziell erhältliche Photonenmeßgeräte, z.B. Photometer oder Photonenzähler, eingesetzt werden. Da sehr geringe Lichtmengen detektiert werden müssen, wird vorteilhaft ein Photonenzähler, insbesondere ein zur Einzelphotonenmessung geeigneter Photonenzähler, eingesetzt. Die Eichung des eingesetzten Photonenzählers kann z.B. in der Weise erfolgen, daß man für die zu analysierende Probe deren Keimzahl nach dem Koch'schen Plattenverfahren und die Biolumineszenz-Lichtmenge nach dem erfindungsgemäßen Verfahren ermittelt und diese beiden Werte miteinander korreliert. Die gemessene BiolumineszenzLichtmenge der zu analysierenden Proben kann auch auf eine ATP-Standardkonzentration bezogen werden, deren Biolumineszenz-Lichtmenge man quantitativ unter identischen Meßbedingungen zuvor ermittelt hat. Vor den eigentlichen Messungen wird zudem der sogenannte Methodenblindwert ermittelt, der ohne zu analysierende Proben für die eingesetzten Lösungen und Reagenzien mit dem verwendeten Photonenmeßgerät gemessen wird. Zur Berechnung der eigentlichen Biolumineszenz-Lichtmenge der zu analysierenden Proben müssen die erhaltenen Meßwerte um den Methodenblindwert korrigiert werden.

Das erfindungsgemäße Verfahren eignet sich besonders zur schnellen Bestimmung der bakteriellen Keimzahl in Molke und Molkeninhaltsstoffen, wie sie in Molkereien oder milchverarbeitenden Betrieben bei der Herstellung von Nahrungsmitteln aus Milch anfallen. In der Regel enthalten die zu analysierenden Proben keine Hefezellen oder somatischen Zellen, die möglicherweise durch ihren ATP-Gehalt die erfindungsgemäße Bestimmung der bakteriellen Keimzahl stören würden. Für den Fall, daß doch nichtbakterielles ATP in den zu analysierenden Proben vorhanden ist, müssen nichtbakterielle Zellen zerstört und das nichtbakterielle ATP abgebaut werden. Hefezellen lassen sich beispielsweise durch bestimmte Antibiotika abtöten. In einer Variante der Erfindung wird nach Durchführung des Verfahrensschrittes a) zunächst eventuell in der zu analysierenden Probe vorhandenes nichtbakterielles ATP abgebaut. Dieser Abbau des nichtbakteriellen ATPs, beispielsweise aus somatischen Zellen oder Hefezellen, kann auf physikalischem, chemischem oder enzymatischem Wege erfolgen. Vorzugsweise setzt man hierfür ein ATP-hydrolysierendes Reagenz, insbesondere ein Enzym, vorzugsweise eine ATPase wie z. B. Somase, ein. Bevor in der zu analysierenden Probe das vorhandene nichtbakterielle ATP durch das ATP-hydrolysierendes Reagenz abgebaut werden kann, muß das ATP zuvor aus den nichtbakteriellen Zellen selektiv freigesetzt werden. Üblicherweise setzt man hierfür ein sogenanntes NRS-Reagenz (NRS = "nucleotid releasing reagent for somatic cells") ein, das im wesentlichen eine wäßrige Lösung mit 2 bis 7 Gew.-% Trichloressigsäure und EDTA in einer Konzentration von 3 bis 4 mg je 1 ml Lösung ist. Bevorzugt sind 5 Gew.-% Trichloressigsäure und EDTA in einer Konzentration von ca. 42 mg je 11,2 ml Lösung enthalten.

In einer bevorzugten Ausführung erfolgt der Aufbruch der nichtbakteriellen Zellen und der Abbau des nichtbakteriellen ATP gleichzeitig, vorzugsweise durch den Zusatz einer Mischung, bestehend aus dem NRS-Reagenz und dem Enzym Somase.

In einer weiteren Variante der Erfindung wird nach Durchführung des Verfahrensschrittes a) die zu analysierende Probe sterilfiltriert, das Filter in eine zur Kultivierung von Bakterien geeignete Nährlösung überführt und nach Inkubation von 1 bis 9 Stunden, insbesondere 1 bis 3 Stunden bei zur Kultivierung von Bakterien geeigneten Temperaturen, beispielsweise zwischen 28 und 38 °C, z.B. bei 35 °C, mit dem Überstand über dem Filter das Verfahren gemäß der Verfahrensschritte b) bis d) durchgeführt. In vorteilhafter Weise wird durch diesen zusätzlichen Verfahrensschritt eine Vermehrung der bakteriellen Keimzahl erreicht, so daß sich mit dem erfindungsgemäßen Verfahren auch geringe bakterielle Kontaminationen in Molke und Molkeninhaltsstoffen nachweisen lassen.

Die Durchführung des erfindungsgemäßen Verfahrens, insbesondere für Lactosepulver, kann zweckmäßigerweise in der Weise erfolgen, daß die zu analysierende Probe in ca. der 8- bis 12-fachen Menge steriler isotonischer Lösung, z.B. Ringerlösung, gelöst wird und in einem Wasserbad für ca. 20 bis 40 Minuten auf 40 bis 60 °C unter leichtem Schütteln erwärmt wird. Anschließend wird durch ein steriles Vakuumfilter filtriert und mit ca. der 5- bis 10-fachen Menge steriler isotonischer Lösung nachgespült. Anschließend wird das Filter in ein steriles Gefäß, z.B. einer Petrischale, überführt und mit einer sterilen bakteriellen Nährlösung überschichtet und für ca. 30 bis 120 Minuten bei 28 bis 38, vorzugsweise bei 35 °C inkubiert. Um aus den Bakterienzellen das bakterielle ATP freizusetzen, wird dann die etwa 0,5- bis 2-fache Menge, bezogen auf die zur Überschichtung des Filters eingesetzte Menge an Nährstofflösung, eines sogenannten NRB-Reagenz (NRB = "nucleotid releasing reagent for microbial cells") hinzugemischt. Als NRB-Reagenz kann beispielsweise eine 0,1 bis 2 %ige wäßrige Lösung eines ionischen Detergenz oder eine wäßrige Lösung, die ca. 1 bis 2 Gew.-% Trichloressigsäure sowie das Enzym Lysozym enthält, verwendet werden. Nach Zugabe des NRB-Reagenz wird für eine gute Durchmischung, beispielsweise durch vorsichtiges Schwenken der Petrischale, gesorgt und nach 1 bis 2 Minuten ein Aliquot von 50 bis 500 µl, vorzugsweise 200 µl, entnommen und in eine durchsichtige Küvette einpipettiert. Die verwendete Küvette sollte für das zu detektierende Biolumineszenz-Licht (562 nm) eine hohe Durchlässigkeit besitzen. Die Küvette wird in ein zur Lumineszenz-Messung geeignetes Photonenmeßgerät eingesetzt und es werden 10 bis 1000 µl, vorzugsweise 100 µl, bekanntes Luciferin-Luciferase-Reagenz hinzugefügt und die in der Probe freiwerdende Biolumineszenz quantitativ gemessen. Aus der gemessenen Biolumineszenz-Lichtmenge wird dann aus der Korrelation zwischen der gemessenen Probe und einem bekannten Standard die Anzahl der bakteriellen Keime in der Probe errechnet.

In identischer Weise, wie für Lactosepulver beschrieben, kann das erfindungsgemäße Verfahren auch zur Bestimmung der Keimzahl in Molkenproteinpulver durchgeführt werden.

Das erfindungsgemäße Verfahren ermöglicht in vorteilhafter Weise die Anwendung der Luciferin-Luciferase-Biolumineszenz-Methode zur Bestimmung der Keimzahl in Molke und Molkeninhaltsstoffen. Unter Molkenprodukten werden dabei insbesondere Lactosepulver und Molkenproteinpulver verstanden, in denen sich schnell und zuverlässig die Keimzahl mit Hilfe des erfindungsgemäßen Verfahrens bestimmen läßt. Gegenüber dem herkömmlichen Plattenverfahren kann das erfindungsgemäße Verfahren wesentlich schneller durchgeführt werden. Auch ob sich Sporenbildner, beispielsweise von bacillus cereus, in den zu analysierenden Proben befinden, läßt sich mit Hilfe des erfindungsgemäßen Verfahrens sehr schnell ermitteln. Bei der Anwesenheit von Sporenbildnern ist die gemessene Biolumineszenz-Lichtmenge deutlich größer als der Methodenblindwert, so daß ab einer bestimmten Biolumineszenz-Lichtmenge, der um einen bestimmten Betrag größer als der Methodenblindwert ist, von der Anwesenheit von Sporenbildnern in den Proben ausgegangen werden kann. Dieser Betrag, um den der Methodenblindwert bei Anwesenheit von Sporenbildnern erhöht ist, muß in Vorversuchen mit Referenz-Proben, die Sporenbildner enthalten ermittelt werden.

Weiterhin umfaßt die Erfindung ein Analyse-Kit für das beschriebene Verfahren zur Bestimmung der bakteriellen Keimzahl in Molke und Molkeninhaltsstoffen, bei dem man in der zu analysierenden Probe einen pH-Wert von 7,0 bis 7,9 einstellt, das bekterielle Adenosintriphosphat durch eine lysierendes Reagenz aus den Bakterienzellen freisetzt, ein Luciferin-Luciferase-Reagenz hinzufügt und die während der Luciferase-Luciferin-Reaktion freigesetzte Biolumeniszenz-Lichtmenge quantitativ bestimmt und aus der ermittelten Lichtmenge die in der Probe enthaltende Menge Adenosintriphosphat bestimmt, wobei der Analyse-Kit
a) ein um den pH-Wert 7,0 bis 8,0 pufferndes Reagenz,
b) ein zur Lysis von Bakterienzellmembranen geeignetes Reagenz,
c) ein Luciferin-Luciferase-Reagenz sowie gegebenenfalls
d) ein Adenosintriphosphat-hydrolysierendes Reagenz enthält.

Bei der Bestimmung der Keimzahl in Lactosepulver oder Molkenproteinpulver wird in dem Analyse-Kit als pufferndes Reagenz eine sterile isotonische Lösung, vorzugsweise sterile physiologische Kochsalzlösung, insbesondere die an sich bekannte Ringerlösung eingesetzt.

Vorteilhaft verwendet man zur Keimzahlbestimmung in Milch oder Molke ein pufferndes Reagenz, welches einen in der biochemischen Analytik üblichen Puffer für den pH-Bereich 7,0 bis 8,0 enthält. Vorzugsweise verwendet man zur Keimzahlbestimmung in Milch oder Molke im Analyse-Kit ein pufferndes Reagenz, welches HEPES-Puffer, insbesondere einen um den pH-Wert 7,5 puffernden HEPES-Puffer enthält. Die Konzentration dieses HEPES-Puffers sollte bevorzugt bei 1 mol/l liegen.

Sollten in der zu analysierenden Probe noch somatische Zellen enthalten sein, müssen diese vor der Bestimmung der bakteriellen Keimzahl zerstört und das nichtbakterielle ATP abgebaut werden. Für diesen Fall enthält das erfindungsgemäße Analyse-Kit gegebenenfalls noch ein ATP-hydrolysierendes Reagenz. Vorzugsweise enthält das ATP-hydrolysierende Reagenz ein Enzym, insbesondere eine ATPase wie z. B. Somase. Soll der Aufbruch der nichtbakteriellen Zellen und der Abbau des nichtbakteriellen ATP gleichzeitig erfolgen, so enthält das ATP-hydrolysierende Reagenz in einer bevorzugten Ausführungsform neben dem Enzym zusätzlich noch eine wäßrige Lösung aus 2 bis 7 Gew.-% Trichloressigsäure und 3 bis 4 mg EDTA je 1 ml Lösung. Bevorzugt enthält diese wäßrige Lösung 5 Gew.-% Trichloressigsäure und EDTA in einer Konzentration von 3,75 mg je 1 ml Lösung.

Mit Hilfe des erfindungsgemäßen Analyse-Kits, welches eine Kombination der vorgenannten Reagenzien darstellt, ist es auf schnelle und reproduzierbare Weise möglich, die bakterielle Keimzahl in Molke und Molkeninhaltsstoffe unter Anwendung der Luciferase-Luciferin-Reaktion zu bestimmen.

Es ist überraschend, daß nach dem angegebenen Verfahren die Bestimmung der bakteriellen Keimzahl in Molke oder Molkeninhaltsstoffen überhaupt möglich ist, da Bakterienzellen weit weniger ATP als beispielsweise somatische Zellen oder Hefezellen enthalten. Es überrascht auch, daß im erfindungsgemäßen Verfahren der Nachweis der Luciferin-Luciferase-Biolumineszenz auch bei geringer bakterieller Keimdichte gelingt. Ein sogenanntes chemisches Quenching, beispielsweise durch die Molkeninhaltsstoffe verursacht, und ein optisches Quenching, hervorgerufen durch die starke Färbung oder Trübung der aus Molke stammenden Proben, tritt praktisch nicht auf. Mit dem erfindungsgemäßen Verfahren sowie dem erfindungsgemäßen Analyse-Kit zur Durchführung dieses Verfahrens wird damit eine Methode zur Verfügung gestellt, mit der unter geringem zeitlichen und auch räumlichen Aufwand schnell und reproduzierbar eine zuverlässige Aussage über die bakterielle Keimbelastung von Molke und Molkeninhaltsstoffen getroffen werden kann.

Die nachfolgenden Beispiele sollen die Erfindung erläutern, ohne sie jedoch in ihrem Umfang zu begrenzen.

### Beispiele

In den nachfolgenden Beispielen wurde zur quantitativen Bestimmung der Biolumineszenz als Photonenzähler der Biocounter M2500 der Firma Lumac Systems, Basel, eingesetzt. Der Biocounter M2500 besitzt eine Bialkali-Photokatode, mit der Licht der Wellenlänge 350 bis 650 nm mit einer maximalen Zählrate von 50.000 Einheiten pro Sekunde gemessen werden kann. Die Eichung des Gerätes erfolgte über einen 200 Pikogramm ATP-Standard unter Bedingungen, wie nachfolgend für die zu analysierenden Proben beschrieben. Vor jeder Meßreihe wurden die Injektionssysteme und die Küvetten mindestens 4 mal mit sterilem destillierten Wasser gespült. Alle Messungen am Gerät wurden unter möglichst sterilen Bedingungen durchgeführt.

Alle eingesetzten Reagenzien, Nährlösungen und Pufferlösungen wurden mit sterilen Medien angesetzt. Nährlösungen wurden vor der Anwendung autoklaviert. Für alle eingesetzten Reagenzien, Puffer und Nährlösungen wurden in Vorversuchen die Biolumineszenz-Blindwerte ermittelt.

### Verwendete Reagenzien, Puffer und Nährlösungen:

### 1. Ringerlösung:

Als Ringerlösung wird eine an sich bekannte isotonische Kochsalzlösung bezeichnet. Diese setzt sich zusammen aus einer wäßrigen Lösung von 0,8 Gew.-% NaCl, 0,02 Gew.-% KCl, 0,02 Gew.-% CaCl₂ und 0,1 Gew.-% NaHCO₃.

### 2. Bakterielle Nährlösung:

Zur Aktivierung der bakteriellen Zelltätigkeit und Anreicherung gering kontaminierter Proben wurde in den hier aufgeführten Beispielen als bakterielle Nährlösung die kommerziell erhältliche Nährlösung mit der Bezeichnung Lumacult^{R} der Firma Lumac Systems AG, Basel, eingesetzt.

### 3. HEPES-Puffer:

Es wurde ein HEPES-Puffer (HEPES = N-2-Hydroxyethylpiperazin-N'-2-ethansulfonsäure) mit einer Konzentration von 1 Mol/l, dessen pH-Wert auf 7,5 eingestellt wurde, verwendet.

### 4. NRB-Reagenz:

Als NRB-Reagenz kann die wäßrige Lösung eines ionischen Detergenz mit einer Konzentration von 0,1 bis 2 Gew.-% eingesetzt werden. In den hier aufgeführten Versuchen wurde das kommerziell erhältliche Reagenz L-NRB^{R} der Firma Lumac Systems, Basel verwendet.

### 5. NRS-Reagenz:

Als NRS-Reagenz kann eine wäßrige Lösung aus 5 Gew.-% Trichloressigsäure und 42 mg/11,2 ml EDTA (EDTA = Ethylendiamintetraessigsäure Dinatriumsalz) eingesetzt werden. In den hier aufgeführten Versuchen wurde das kommerziell erhältliche Reagenz NRS^{R} der Firma Lumac Systems, Basel, verwendet.

### 6. Somase-Lösung:

Es wurde kommerziell erhältliche gefriergetrocknete Somase eingesetzt. Die Somase wurde vor der Verwendung mit einem entsprechenden Puffer rekonstruiert. Hierfür wurde der kommerziell unter dem Namen Lumit^{R} von der Firma Lumac Systems AG, Basel, erhältliche Puffer verwendet. Zu 1 ml Somase-Lösung wurden 5 ml NRS^{R}-Lösung gegeben.

### 7. Luciferin-Luciferase-Reagenz:

Leuchtkäfer-Luciferin-Luciferase-Reagenz ist in gefriergetrockneter Form kommerziell erhältlich, wobei die kommerziell erhältlichen Produkte zumeist noch Rinderserum-Albumin sowie Dithiothreitol enthalten. Für die in den Beispielen beschriebenen Messungen wurde das Luciferin-Luciferase-Reagenz, das unter dem Handelsnamen Lumit-PM^{R} von der Firma Lumac Systems AG, Basel erhältlich ist, eingesetzt. Die gebrauchsfertige Lösung des Luciferin-Luciferase-Reagenz wurde hergestellt, indem das gefriergetrocknete Luciferin-Luciferase-Reagenz in einem Puffer mit einem pH-Wert von 7,75 gelöst wurde. Dieser Puffer setzte sich zusammen aus 25 mM HEPES, 7,5 mM Magnesiumsulfat und 1 mM EDTA. In den hier angegebenen Beispielen wurde der kommerziell unter dem Namen Lumit^{R} von der Firma Lumac Systems AG, Basel, erhältliche Puffer eingesetzt.

Im Analyse-Kit in den nachfolgenden Beispielen I., III., und IV. wurde neben dem Luciferin-Luciferase-Reagenz als pufferndes Reagenz Ringerlösung und als zur Lysis von Bakterienzellmembranen geeignetes Reagenz das obenbeschriebene NRB-Reagenz eingesetzt. In Beispiel II. wurde als pufferndes Reagenz der obenbeschriebene HEPES-Puffer verwendet. Die Analyse-Kits der Beispiele II. bis IV. enthielten zusätzlich noch als ATP-hydrolysierendes Reagenz die obenbeschriebene Somase-Lösung.

### I. Messung der bakteriellen Keimzahl in Lactosepulver

In 99 ml mit sterilem destilliertem Wasser verdünnte Ringerlösung (0,25-fach konzentriert) wurden 11 g Lactosepulver steril eingewogen, die Probenlösung mehrfach geschüttelt und in ein Wasserbad mit einer Temperatur von ca. 50 °C für 30 Minuten erwärmt. Innerhalb dieser Zeit wurde die Probe mehrmals bewegt. Anschließend wurde die Lösung warm durch ein steriles Vakuumfilter (Porendurchmesser 0,45 µm, Pall-positiv-Filter) filtriert und mit ca. 50 ml Ringerlösung nachgespült. Das Filter wurde in eine sterile Petrischale gelegt und mit 0,5 ml bakterieller Nährlösung vollständig überschichtet. Nachdem die Petrischale bei ca. 35 °C für 80 Minuten inkubiert worden war, wurden 0,5 ml NRB-Reagenz zugegeben. Anschließend wurde die Schale noch ca. 90 Sekunden vorsichtig geschwenkt. Zur besseren Vermischung wurden 200 µl des Filterüberstandes 3 mal mit einer sterilen Pipette aufgezogen und wieder abgelassen. Anschließend wurden 200 µl der Probe in eine Küvette eingefüllt und in den Photonenzähler eingesetzt. Über das Injektionssystem des Photonenzählers wurden automatisch 100 µl Luciferin-Luciferase-Reagenz zugegeben und die Biolumineszenz-Lichtmenge gemessen. Die gemessene Lichtmenge wurde vom Photonenzähler nach 10 Sekunden Integrationszeit als RLU-Wert ausgegeben (RLU = relative Lichteinheiten). Aus den gemessenen RLU-Werten konnte in Relation zum RLU-Wert des ATP-Standards die in der Probe vorhandene ATP-Menge errechnet werden.

Zur Ermittlung des Biolumineszenz-Blindwertes wurde die gesamte Messung noch einmal unter identischen Bedingungen ohne Lactosepulver durchgeführt.

### II. Bestimmung der bakteriellen Keimzahl in Molke

0,5 ml Molke wurden in eine Küvette gegeben und 0,4 ml HEPES-Puffer und 100 µl einer Lösung, die Somase im NRS-Reagenz enthielt, zugegeben. Bei Raumtemperatur wurde anschließend für ca. 20 Minuten auf einem Schüttler (Model IKA-Vibrax-VXR) geschüttelt. Mit einer sterilen Pipette wurden 100 µl dieser Lösung in eine weitere Küvette gefüllt und anschließend die Küvette in den Photonenzähler eingesetzt. Über das Injektionssystem des Photonenzählers wurden automatisch 100 µl NRB-Reagenz und anschließend 100 µl Luciferin-Luciferase-Reagenz zugegeben und die Biolumineszenz-Lichtmenge gemessen. Nach 60 Sekunden Integrationszeit wurde ein RLU-Wert ausgegeben, aus dem in Relation zum RLU-Wert des ATP-Standards die in der Probe vorhandene ATP-Menge errechnet wurde.

Der Biolumineszenz-Blindwert wurde wie unter Beispiel I. beschrieben, ermittelt.

### III. Bestimmung der bakteriellen Keimzahl in Molkenproteinpulver

In 99 ml Ringerlösung wurden 11 g Molkenproteinpulver eingewogen. Bei 20 °C wurde diese Lösung für 20 Minuten stehengelassen und mehrfach geschüttelt. Mit einer sterilen Pipette wurden 0,5 ml Lösung entnommen und in eine Küvette überführt. Nach Zugabe von 100 µl Lösung, die Somase im NRS-Reagenz enthielt, und 0,4 ml HEPES-Puffer wurde für 40 Minuten bei Raumtemperatur inkubiert. Anschließend wurden 100 µl mit einer sterilen Pipette in eine weitere Küvette überführt, und diese Küvette in den Photonenzähler eingesetzt. Nach Zugabe von 100 µl NRB-Reagenz und anschließender Zugabe von 100 µl Luciferin-Luciferase-Reagenz über das automatische Injektionssystem des Photonenzählers wurde die freigesetzte Biolumineszenz-Lichtmenge gemessen. Nach 60 Sekunden Integrationszeit wurde ein RLU-Wert angegeben, aus dem in Relation zum RLU-Wert des ATP-Standards die in der Probe vorhandene ATP-Menge errechnet wurde.

### IV. Nachweis von Sporenbildnern in Lactosepulver

11 g Lactosepulver wurden in 99 ml Ringerlösung gelöst und unter Schütteln für 12 Minuten bei 85 °C im Wasserbad erhitzt. Anschließend wurde die Lösung steril durch ein Vakuumfilter (Porendurchmesser 0,45 µm, Pall-Positiv-Filter) filtriert. Das Filter wurde anschließend in einer sterilen Petrischale mit 0,5 ml bakterieller Nährlösung vollständig überschichtet und für ca. 6 Stunden bei 35 °C inkubiert. Nach Zugabe von 40 µl Somase wurde für 1 Stunde bei Raumtemperatur inkubiert. Anschließend wurden 0,5 ml NRB-Reagenz hinzugegeben und die Petrischale noch für ca. 60 Sekunden vorsichtig geschüttelt. Mit einer sterilen Pipette wurden 200 µl des Filterüberstandes in eine Küvette überführt, und diese Küvette in den Photonenzähler eingesetzt. Über das automatische Injektionssystem des Photonenzählers wurden 100 µl Luciferin-Luciferase-Reagenz hinzugegeben und die Biolumineszenz-Lichtmenge ermittelt. Nach einer Integrationszeit von 10 Sekunden wurde der RLU-Wert ermittelt. Ab einem RLU-Wert, der um 20 RLU-Werteinheiten größer als der Methodenblindwert war, konnte von der Anwesenheit von Sporenbildnern ausgegangen werden.

Der Blindwert wurde, wie bereits unter Beispiel I beschrieben, ermittelt.

## Patentansprüche

1. Verfahren zur Bestimmung der bakteriellen Keimzahl in Molke und Molkeninhaltsstoffen, dadurch gekennzeichnet, daß man in der zu analysierenden Probe
a) einen pH-Wert von 7,0 bis 7,9 einstellt,
b) das bakterielle Adenosintriphosphat durch ein lysierendes Reagenz aus den Bakterienzellen freisetzt,
c) eine Luciferin-Luciferase-Reagenz hinzufügt und
d) die während der Luciferase-Luciferin-Reaktion freigesetzte Biolumineszenz-Lichtmenge quantitativ bestimmt und aus der ermittelten Lichtmenge die in der Probe enthaltende Menge Adenosintriphosphat bestimmt.

2. Verfahren nach Anspruch 1, dadurch gekennzeichnet, daß man nach Durchführung des Verfahrensschrittes a) die zu analysierende Probe sterilfiltriert, das Filter in eine zur Kultivierung von Bakterien geeignete Nährlösung überführt und nach einer Inkubation für 1 bis 9 Stunden bei 28 bis 38 °C mit dem Überstand über dem Filter das Verfahren gemäß der Verfahrensschritte b) bis d) durchführt.

3. Verfahren nach Anspruch 1, dadurch gekennzeichnet, daß man im Verfahrenschritt a) einen pH-Wert von 7,3 bis 7,5 einstellt.

4. Verfahren nach Anspruch 1, dadurch gekennzeichnet, daß man vor Durchführung der Verfahrensschritte b) bis d) zunächst nichtbakterielles Adenosintriphosphat in der zu analysierenden Probe durch ein Adenosintriphosphat-hydrolysierendes Reagenz abbaut.

5. Verfahren nach Anspruch 1 bis 4, dadurch gekennzeichnet, daß man das Verfahren zur Bestimmung der bakteriellen Keimzahl in Lactosepulver durchführt.

6. Verfahren nach Anspruch 1 bis 4, dadurch gekennzeichnet, daß man das Verfahren zur Bestimmung der Keimzahl in Molkenproteinpulver durchführt.

7. Analyse-Kit für ein Verfahren zur Bestimmung der bakteriellen Keimzahl in Molke und Molkeninhaltsstoffen, bei dem man in der zu analysierenden Probe einen pH-Wert von 7,0 bis 7,9 einstellt, das bakterielle Adenosintriphosphat durch ein lysierendes Reagenz aus den Bakterienzellen freisetzt, ein Luciferin-Luciferase-Reagenz hinzufügt und die während der Luciferase-Luciferin-Reaktion freigesetzte Biolumineszenz-Lichtmenge quantitativ bestimmt und aus der ermittelten Lichtmenge die in der Probe enthaltende Menge Adenosintriphosphat bestimmt, dadurch gekennzeichnet, daß der Analyse-Kit
a) ein um den pH-Wert 7,0 bis 8,0 pufferndes Reagenz,
b) ein zur Lysis von Bakterienzellmembranen geeignetes Reagenz,
c) ein Luciferin-Luciferase-Reagenz sowie gegebenenfalls
d) ein Adenosintriphosphat-hydrolysierendes Reagenz enthält.

8. Analyse-Kit nach Anspruch 7, dadurch gekennzeichnet, daß es als pufferndes Reagenz Ringerlösung enthält.

9. Analyse-Kit nach Anspruch 7, dadurch gekennzeichnet, daß es als pufferndes Reagenz einen um den pH-Wert 7,5 pufferndes HEPES-Puffer enthält.

10. Analyse-Kit nach Anspruch 7, dadurch gekennzeichnet, daß es als Adenosintriphosphat-hydrolysierendes Reagenz eine ATPase enthält.

11. Analyse-Kit nach Anspruch 10, dadurch gekennzeichnet, daß das Adenosintriphosphat-hydrolysierende Reagenz eine wäßrige Lösung aus 2 bis 7 Gew.-% Trichloressigsäure und 3 bis 4 mg EDTA je 1 ml Lösung enthält.
